# EUROPEAN PATENT SPECIFICATION

(11) **EP 2 068 817 B1**
(45) Date of publication and mention of the grant of the patent: **23.03.2016**
(21) Application number: 07839093.7
(22) Date of filing: 27.09.2007
(51) Int. Cl.: A61K 8/37, A61K 8/40, A61Q 17/04

(54) **PHOTOPROTECTIVE COMPOSITIONS COMPRISING SYNERGISTIC COMBINATION OF SUNSCREEN ACTIVE COMPOUNDS**
PHOTOPROTEKTIVE ZUSAMMENSETZUNGEN MIT EINER SYNERGISTISCHEN KOMBINATION AUS WIRKSAMEN SONNENSCHUTZVERBINDUNGEN
COMPOSITIONS PHOTOPROTECTRICES COMPRENANT UNE COMBINAISON SYNERGIQUE DE COMPOSÉS ACTIFS DE PROTECTION SOLAIRE

(30) Priority: 02.10.2006 US 541763
(43) Date of publication of application: 17.06.2009
(73) Proprietor: Bayer Consumer Care Holdings LLC, Whippany, NJ 07950 (US)
(72) Inventor: BEASLEY, Donathan, G., Memphis, Tennessee 38128 (US); MEYER, Thomas, A., Germantown, Tennessee 38139 (US)
(74) Representative: BIP Patents
(86) International application number: PCT/US2007/021067
(87) International publication number: WO 2008/042326

(56) References cited:
- EP-A- 1 310 238
- EP-A- 1 591 104
- WO-A-02/28359
- WO-A-2007/128840
- DE-A1- 19 820 660
- FR-A- 2 802 417
- US-B1- 6 440 402

## Description

### FIELD OF THE INVENTION

The subject invention is directed to compositions for topical application containing a synergistic combination of sunscreen active compounds, and more particularly to compositions for topical application comprising a synergistic combination of octinoxate, octocrylene, and amiloxate.

### BACKGROUND OF THE INVENTION

When applied topically, sunscreen compositions impart a film that protects skin against the damaging effects of exposure to the sun's ultraviolet radiation (UVR). Sunscreen active compounds work on skin's surface by absorbing UVR before it can interact with and damage skin. A particular composition's ability to block UVR is usually expressed in a sun protection factor or SPF rating. Combinations of sunscreen active compounds are typically used in sunscreen composition in order to raise the SPF rating of the composition.

U.S. Patent Nos. 6,030,629 and 5,968,481 assert synergistic increase in SPF based on combinations of certain sunscreen active compounds. Further, André et al. report a synergistic effect.based on a combination of organic and inorganic sunscreens covering UV-A and UV-B absorption. (V. André et al., Cosmetics and Toiletries Manufacture Worldwide 2004, pp. 19-23.) However, as those authors note, such synergistic increases in SPF are not completely understood, nor is it predictable before hand which combination of compounds will demonstrate a synergistic effect. In fact, as demonstrated in the experiments herein, a combination of only two of the three sunscreen actives in the present invention does not show synergistic SPF increases.

Thus a combination of sunscreen active compounds that demonstrate a synergistic increase in SPF, and thus an enhanced UVR protectective capability over what would be predicted from their use alone, would be useful and desirable.

These and other objectives are obtained by the invention more fully described and claimed herein.

### SUMMARY OF THE INVENTION

The subject invention provides a topically applicable sunscreen composition suited for the photoprotection of human skin and/or hair, comprising photoprotecting synergistically effective amounts of octinoxate, octocrylene, and amiloxate, as defined in claim 1.

The subject invention further provides a composition for use in a method for protecting human skin and/or hair against the deleterious effects of solar radiation, more particularly ultraviolet radiation (UVR) comprising topically applying thereto an effective amount of the sunscreen compositions as described herein.

### DETAILED DESCRIPTION

The subject invention provides a topically applicable sunscreen composition suited for the photoprotection of human skin and/or hair, comprising photoprotecting synergistically effective amounts of octinoxate, octocrylene, and amiloxate. Octinoxate, which is also known as octyl methoxycinnamate or ethylhexyl methoxycinnamate, is listed in Annex VII of the EU Cosmetics Directive for use as a sunscreen agent up to a level of 10%. In the United States, it is listed by the USFDA for use at level up to 7.5%. Octocrylene, whose chemical name is 2-ethylhexyl 2-cyano-3,3-diphenyl-2-propenoate, is listed by the USFDA for use up to 10% and in the EU Cosmetics Directive for use up to 10%. Amiloxate, whose chemical name is 2-benzoic acid, 2-propenoic acid,3-(4-methoxyphenyl)-3-methylbutyl ester and is also known by 4-Methoxycinnamic acid,isoamyl ester or isoamyl p-methoxycinnamate, is being reviewed for approval by the USFDA for use up to 10% and is listed in the EU Cosmetics Directive for use up to 10%. It is understood that the compositions of the invention are not limited to these statutory limits but that these limits are provided to illustrate practical limitations on the amount of these sunscreen active agents in products that may be marketed in various countries

In one embodiment of the invention, the molar ratio of octinoxate:octocrylene:amiloxate present in the composition may range from .034:.028:.04 to 0.26:0.28:0.40.

Octinoxate is present in the composition an amount from about 6.5 to about 7.5 percent by in an amount from about 6.5 to about 7.5 percent by weight.

Octocrylene is present in the composition in an amount from about 5.0 to about 10.0 percent by weight, more preferably in an amount from about 8.0 to about 10.0 percent by weight.

Amiloxate is present in the composition in an amount from about 5.0 to about 10.0 percent by weight, more preferably in an amount from about 7.5 to about 10.0 percent by weight.

The compositions of the invention containing the synergistic combination of sunscreen actives can comprise any form readily known by those of ordinary skill in the art of preparing cosmetic compositions, in particular compositions containing sunscreen active agents. Examples of such include, but are not limited to, nonionic vesicle dispersions, emulsions, creams, milks, gels, cream gels, ointments, suspensions, dispersions, powders, solids, sticks, foams or sprays.
In certainly preferred embodiments, the composition can comprise an anhydrous or aqueous solid or paste, emulsion, suspension, or dispersion. Preferable forms of the compositions include an oil-in-water emulsion, a water-in-oil emulsion, an alcohol solution, or an aerosol formulation.

In one embodiment the subject invention, the composition can be in the form of an aerosol, wherein the composition is combined with at least one propellant, which may be any suitable gas that can be compressed or liquefied within a spray dispensing canister and which expands or volatilizes to vapor or gas form upon exposure to ambient temperature and pressure conditions to deliver the composition in an aerosol form. Suitable propellants include hydrocarbons having 1 to 5 carbon atoms, including but not limited to methane, ethane, propane, isopropane, butane, isobutane, butene, pentane, isopentane, neopentane, pentene, hydrofluorocarbons (HFCs), chlorofluorocarbons(CFCs), nitrogen, ethers including dimethyl ether, and any mixtures thereof. Those of ordinary skill in the art recognize that in a closed container such as an aluminum can or glass bottle, propellants such as dimethyl ether condense to the liquid state at ambient temperature. Thus, the composition in the aerosol container is liquid formulation which can contain dissolved propellant, undissolved liquid propellant and gaseous propellant. All of this is under pressure due to the vapor pressure of the propellant. In the practice of the subject invention, the propellant can be present in an amount up to about 90 weight percent, preferably from about 2 weight percent to about 50 weight percent, and more preferably about 5 weight percent to about 40 weight percent, most preferably 30 weight percent, based on the total weight of the aerosol composition.

The compositions of the invention can also comprise aerosol foams or so-called mousse compositions. For example, U.S. Patent No. 6,627,585 describes a mousse-forming cleansing shampoo composition comprising a foamable concentrate comprising at least one surfactant, dispersed particles of a water-insoluble conditioning agent, an aqueous carrier; and an aerosol propellant. U.S. Patent No. 6,264,964 describes a cosmetic composition including a crosslinked non-emulsifying polysiloxane elastomer and a carboxyvinyl polymer which is in the form of an aerosol foam in a pressurized system. The propellant may be introduced into the mousse composition at the time of filling by using a standard aerosol dispenser, e.g. a spray can arrangement.

The subject invention contemplates the incorporation of the synergistic combination of sunscreen actives in sunscreen and sunblock products and any other topically applied composition where the addition of sunscreen active agents would not detract from the efficacy of the product nor affect the sunscreening ability of the sunscreen active agents. Thus the subject invention also provides a cosmetic composition for topical application to human skin and/or hair, comprising photoprotecting synergistically effective amounts of octinoxate, octocrylene, and amiloxate. Non-limiting examples of such cosmetic compositions may include such products as moisturizers, cleansers, conditioners, shampoo, body wash, styling gel/lotion, eye cream and eye liner, blush, mascara, foundation, nail polish, polish remover, eye shadow, lipstick, lip gloss, lip liners, lip balms, makeup remover, nail treatment, foot care compositions, acne treatment, redness/rosacea treatment, varicose/spider vein treatment, anti-aging compositions, sunless tanning compositions, after-sun compositions, concealers, hair color and bleaching compositions, skin fading/lighteners, body firming lotion, shaving cream, after shave, relaxer, antiperspirants and deodorants, exfoliants, scrubs, liquid hand soap, bubble bath, pain and wound treatment compositions, insect repellant, anti-itch and rash cream, styling mousse and foams, perfume, lubricants, body oil, body spray, baby lotion, diaper cream, baby soap, baby shampoo, baby oil, baby wipes, hair-loss treatment, hair spray, depilatory, hair growth inhibitors, hair removal waxes, personal cleansing, cologne, oil controller, and hand sanitizer.

The compositions of the present invention may contain a wide range of additional, optional components which are referred to herein as "cosmetic components", but which can also include components generally known as pharmaceutical active agents. The CTFA Cosmetic Ingredient Handbook, Seventh Edition, 1997 and the Eighth Edition, 2000, describes a wide variety of cosmetic and pharmaceutical ingredients commonly used in skin care compositions, which are suitable for use in the compositions of the present invention. Examples of these functional classes disclosed in this reference include: absorbents, abrasives, anticaking agents, antifoaming agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, plasticizers, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), sunscreen agents, ultraviolet light absorbers, SPF boosters, waterproofing agents, and viscosity increasing agents (aqueous and nonaqueous).

In the practice of the invention, the composition may contain one or more additional sunscreen active agents. For purposes of the present invention, a "sunscreen active agent" or "sunscreen active" shall include all of those materials, singly or in combination, that are regarded as acceptable for use as active sunscreening ingredients based on their ability to absorb UV radiation. Such compounds are generally described as being UV-A, UV-B, or UV-A/UV-B active agents. Approval by a regulatory agency is generally required for inclusion of active agents in formulations intended for human use. Those active agents which have been or are currently approved for sunscreen use in the United States include organic and inorganic substances including, without limitation, para aminobenzoic acid, avobenzone, cinoxate, dioxybenzone, homosalate, menthyl anthranilate, octyl salicylate, oxybenzone, padimate O, phenylbenzimidazole sulfonic acid, sulisobenzone, trolamine salicylate, titanium dioxide, zinc oxide, diethanolamine methoxycinnamate, digalloy trioleate, ethyl dihydroxypropyl PABA, glyceryl aminobenzoate, lawsone with dihydroxyacetone, red petrolatum. Examples of additional sunscreen actives that have not yet been approved in the US but are allowed in formulations sold outside of the US include ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidenecamphor, and isopentyl 4-methoxycinnamate. However, as the list of approved sunscreens is currently expanding, those of ordinary skill will recognize that the invention is not limited to sunscreen active agents currently approved for human use but is readily applicable to those that may be allowed in the future.

In one embodiment of the invention the additional sunscreen active agent comprises a photoprotecting effective amount of particulates of at least one inorganic pigment or nanopigment, non-limiting examples of which include titanium dioxide, zinc oxide, iron oxide, zirconium oxide, cerium oxide, or mixture thereof.

The compositions of the invention may also include materials that also increase the SPF of the final composition by such mechanisms as UV radiation scattering and dispersion. Such materials are referred to herein as "UV-radiation scattering agents" and comprise materials that exhibit UV absorbing activity or exhibit no UV absorbing activity. An example of such UV-radiation scattering agents include polymeric materials, such as the product known as SunSpheres™ (Rohm and Haas; Philadelphia, PA) which are described by their manufacturer as hollow styrene/acrylates copolymer spheres manufactured by emulsion polymerization. The polymer spheres are said to raise SPF values across the UVA and UVB region by dispersing and/or scattering the incident UV radiation throughout the film of sunscreen present on a surface, such as human skin. It is understood that the spheres cause less UV radiation to penetrate into the skin by redirecting the radiation towards the UV-absorbing sunscreen actives in the sunscreen formulation, where the radiation reacts with the sunscreen active molecules and the energy is dissipated as heat. As used herein, the terms "spheres" or "scattering agents" are not limited by chemical makeup or shape, but comprise any agent that produces the effect of lengthening the path of incident UV radiation, increasing the statistical likelihood that the radiation will contact a sunscreen active molecule, i.e., a UV absorbing active agent. These materials may also include UV absorbing materials that also exhibit scattering properties such as ZnO (examples include Z-Cote™ products available from BASF), TiO₂ (examples include the Solaveil™ products available from Uniqema (New Castle, DE, USA)), compounds such as methylene bis-benzotriazolyl tetramethylbutylphenol, ("Tinasorb™ M" available from Ciba Specialty Chemicals, Inc. (Basel, Switzerland). UV radiation scattering agents are typically present in the formulation in amounts up to about 10% by weight, preferably in ranges of about 0.5% to about 7.0% by weight, in particularly preferred ranges of 3% to about 5% by weight.

As used herein, the terms "sunless-tanning agent" or "self-tanning compositions" refer to compositions which, when applied to human skin, impart thereto an appearance similar to that achieved by exposing the skin to natural or artificial sunlight. Examples of sunless tanning active agents are described in U.S. Patent Nos. 6,482,397, 6,261,541, and 6,231,837. Such sunless tanning compositions typically comprise, in addition to an artificial tanning effective amount of a self tanning agent, effective amounts of a composition coloring agent and a cosmetically acceptable carrier adapted for topical application to human skin. The self tanning agents can also include those compositions generally accepted in the art for application to human skin, and which, when so applied, react therein with amino acids so as to form pigmented products. Such reactions give the skin a brown appearance similar to the color obtained upon exposing it to sunlight for periods of time sufficient to tan the skin. Suitable self tanning agents include, without limitation, alpha-hydroxy aldehydes and ketones, glyceraldehyde and related alcohol aldehydes, various indoles, imidazoles and derivatives thereof, and various approved pigmentation agents. Presently preferred herein as self tanning agents are the alpha-hydroxy aldehydes and ketones. Most preferably, the self tanning agent is dihydroxyacetone ("DHA"). Other suitable self tanning agents include, without limitation, methyl glyoxal, glycerol aldehyde, erythrulose, alloxan, 2,3-dihydroxysuccindialdehyde, 2,3-dimethoxysuccindialdehyde, 2-amino-3-hydroxy-succindialdehyde and 2-benzylamino-3-hydroxysuccindialdehyde.

Suitable emulsifiers or surfactants include pharmaceutically acceptable, non-toxic, non-ionic, anionic and cationic surfactants. Examples of suitable non-ionic surfactants include glycerol fatty acid esters such as glycerol monostearate, glycol fatty acid esters such as propylene glycol monostearate, polyhydric alcohol fatty acid esters such as polyethylene glycol (400) monooleate, polyoxyethylene fatty acid esters such as polyoxyethylene (40) stearate, polyoxyethylene fatty alcohol ethers such as polyoxyethylene (20) stearyl ether, polyoxyethylene sorbitan fatty acid esters such as polyoxyethylene sorbitan monostearate, sorbitan esters such as sorbitan monostearate, alkyl glycosides such as cetearyl glucoside, fatty acid ethanolamides and their derivatives such as the diethanolamide of stearic acid, and the like. Examples of suitable anionic surfactants are soaps including alkali soaps, such as sodium, potassium and ammonium salts of aliphatic carboxylic acids, usually fatty acids, such as sodium stearate. Organic amine soaps include organic amine salts of aliphatic carboxylic acids, usually fatty acids, such as triethanolamine stearate. Metallic soaps include salts of polyvalent metals and aliphatic carboxylic acids, usually fatty acids, such as aluminium stearate. Other classes of suitable anionic surfactants include sulfated fatty acid alcohols such as sodium lauryl sulfate, sulfated oils such as the sulfuric ester of ricinoleic acid disodium salt, and sulfonated compounds such as alkyl sultonates including sodium cetane sulfonate, amide sulfonates such as sodium N-methyl-N-oleyl laurate, sulfonated dibasic acid esters such as sodium dioctyl sulfosuccinate, alkyl aryl sulfonates such as sodium dodecylbenzene sulfonate, alkyl naphthalene sulfonates such a sodium isopropyl naphthalene sulfonate, petroleum sulfonate such as aryl napthalene with alkyl substitutes. Examples of suitable cationic surfactants include amine salts such as octadecyl ammonium chloride, quartemary ammonium compounds such as benzalkonium chloride.

An emollient is an oleaginous or oily substance which helps to smooth and soften the skin, and may also reduce its roughness, cracking or irritation. Typical suitable emollients include mineral oil having a viscosity in the range of 50 to 500 centipoise (cps), lanolin oil, coconut oil, cocoa butter, olive oil, almond oil, macadamia nut oil, aloe extracts such as aloe vera lipoquinone, synthetic jojoba oils, natural sonora jojoba oils, safflower oil, corn oil, liquid lanolin, cottonseed oil and peanut oil. Preferably, the emollient is a cocoglyceride, which is a mixture of mono, di and triglycerides of cocoa oil, sold under the trade name of Myritol 331 from Henkel KGaA, or Dicaprylyl Ether available under the trade name Cetiol OE from Henkel KGaA or a C₁₂-C₁₅ Alkyl Benzoate sold under the trade name Finsolv TN from Finetex. One or more emollients may be present ranging in amounts from about 1 percent to about 10 percent by weight, preferably about 5 percent by weight. Another suitable emollient is DC 200 Fluid 350, a silicone fluid, available Dow Corning Corp.

Other suitable emollients include squalane, castor oil, polybutene, sweet almond oil, avocado oil, calophyllum oil, ricin oil, vitamin E acetate, olive oil, silicone oils such as dimethylopolysiloxane and cyclomethicone, linolenic alcohol, oleyl alcohol, the oil of cereal germs such as the oil of wheat germ, isopropyl palmitate, octyl palmitate, isopropyl myristate, hexadecyl stearate, butyl stearate, decyl oleate, acetyl glycerides, the octanoates and benzoates of (C₁₂-C₁₅) alcohols, the octanoates and decanoates of alcohols and polyalcohols such as those of glycol and glyceryl, ricinoleates esters such as isopropyl adipate, hexyl laurate and octyl dodecanoate, dicaprylyl maleate, hydrogenated vegetable oil, phenyltrimethicone, jojoba oil and aloe vera extract.

Other suitable emollients which are solids or semi-solids at ambient temperatures may be used. Such solid or semi-solid cosmetic emollients include glyceryl dilaurate, hydrogenated lanolin, hydroxylated lanolin, acetylated lanolin, petrolatum, isopropyl lanolate, butyl myristate, cetyl myristate, myristyl myristate, myristyl lactate, cetyl alcohol, isostearyl alcohol and isocetyl lanolate. One or more emollients can optionally be included in the formulation.

A humectant is a moistening agent that promotes retention of water due to its hygroscopic properties. Suitable humectants include glycerin, polymeric glycols such as polyethylene glycol and polypropylene glycol, mannitol and sorbitol. Preferably, the humectant is Sorbitol, 70% USP or polyethylene glycol 400, NF. One or more humectants can optionally be included in the formulation in amounts from about 1 percent to about 10 percent by weight, preferably about 5 percent by weight.

A dry-feel modifier is an agent which when added to an emulsion, imparts a "dry feel" to the skin when the emulsion dries. Dry feel modifiers can include talc, kaolin, chalk, zinc oxide, silicone fluids, inorganic salts such as barium sulfate, surface treated silica, precipitated silica, fumed silica such as an Aerosil available from Degussa Inc. of New York, N.Y. U.S.A. Another dry feel modifier is an epichlorohydrin cross-linked glyceryl starch of the type that is disclosed in U.S. Patent No. 6,488,916.

It may be advantageous to incorporate additional thickening agents, such as, for instance, various Carbopols available from Noveon Co. Particularly preferred are those agents which would not disrupt the lamellar structure in the formulation of the final product, such as non-ionic thickening agents. The selection of additional thickening agents is well within the skill of one in the art.

An "antioxidant" is a natural or synthetic substance added to the sunscreen to protect from or delay its deterioration due to the action of oxygen in the air (oxidation). They may also reduce oxidation reactions in skin tissue. Anti-oxidants prevent oxidative deterioration which may lead to the generation of rancidity and nonenyzymatic browning reaction products. Typical suitable antioxidants include propyl, octyl and dodecyl esters of gallic acid, butylated hydroxyanisole (BHA, usually purchased as a mixture of ortho and meta isomers), butylated hydroxytoluene (BHT), green tea extract, uric acid, cysteine, pyruvate, nordihydroguaiaretic acid, Vitamin A, Vitamin E and Vitamin C and their derivatives. One or more antioxidants can optionally be included in the sunscreen composition in an amount ranging from about 0.001 to about 5 weight percent, preferably about 0.01 to about 0.5 percent.

"Chelating agents" are substances used to chelate or bind metallic ions, such as with a heterocylic ring structure so that the ion is held by chemical bonds from each of the participating rings. Suitable chelating agents include ethylene diaminetetraacetic acid (EDTA), EDTA disodium, calcium disodium edetate, EDTA trisodium, albumin, transferrin, desferoxamine, desferal, desferoxamine mesylate, EDTA tetrasodium and EDTA dipotassium, or combinations of any of these.

"Fragrances" are aromatic substances which can impart an aesthetically pleasing aroma to the sunscreen composition. Typical fragrances include aromatic materials extracted from botanical sources (i.e., rose petals, gardenia blossoms, jasmine flowers, etc.) which can be used alone or in any combination to create essential oils. Alternatively, alcoholic extracts may be prepared for compounding fragrances. However, due to the relatively high costs of obtaining fragrances from natural substances, the modern trend is to use synthetically prepared fragrances, particularly in high-volume products. One or more fragrances can optionally be included in the sunscreen composition in an amount ranging from about 0.001 to about 5 weight percent, preferably about 0.01 to about 0.5 percent by weight. Additional preservatives may also be used if desired and include well known preservative compositions such as benzyl alcohol, phenyl ethyl alcohol and benzoic acid, diazolydinyl, urea, chlorphenesin, iodopropynyl and butyl carbamate, among others.

The compositions of the invention can further comprise skin protectant active agents. Suitable examples include (with preferred weight percent ranges), Allantoin (0.5 to 2 percent); Aluminum hydroxide gel (0.15 to 5 percent); Calamine (1 to 25 percent); Cocoa butter (greater than 50); Cod liver oil (5 to 14 percent); Colloidal oatmeal; Dimethicone (1 to 30 percent); Glycerin (20 to 45 percent); Hard fat (greater than 50); Kaolin (4 to 20 percent); Lanolin (12.5 to 50 percent); Mineral oil (greater than 50 percent); Petrolatum (greater than 30 percent); Sodium bicarbonate; Topical starch (10 to 98 percent); White petrolatum (greater than 30 percent); Zinc acetate (0.1 to 2 percent); Zinc carbonate (0.2 to 2 percent); and Zinc oxide (1 to 25 percent).

The compositions of the invention may further include insect repelling components. The most widely used insect repelling active agent for personal care products is N,N-Diethyl-m-toluamide, frequently called "DEET" and available in the form of a concentrate containing at least about 95 percent DEET. Other synthetic chemical repellents include ethyl butylacetylaminoproprionate (also known as IR 3535), dimethyl phthalate, ethyl hexanediol, indalone, di-n-propylisocinchoronate, bicycloheptene, dicarboximide and tetrahydrofuraldehyde. Certain plant-derived materials also have insect repellent activity, including citronella oil and other sources of citronella (including lemon grass oil), limonene, rosemary oil and eucalyptus oil. Choice of an insect repellent for incorporation into the sunscreen emulsion will frequently be influenced by the odor of the repellent. The amount of repellent agent used will depend upon the choice of agent; DEET is useful at high concentrations, such as up to about 15 percent or more, while some of the plant-derived substances are typically used in much lower amounts, such as 0.1 percent or less.

Topical application of the compositions of the invention described herein to the hair or skin of a human will provide enhanced protection against deleterious effects of ultraviolet radiation (UVR). Thus, the subject invention further provides a method for protecting human skin and/or hair against the deleterious effects of solar radiation, more particularly UVR, which method comprises topically applying thereto an effective amount of the sunscreen compositions as described herein. An esthetically beneficial result of exposure of skin to UVR (i.e., light radition wavelengths of from 280 nm to 400 nm) is the promotion of tanning of the human epidermis. Another benefit of sun exposure comes from production of vitamin D within the skin. UVR is typically divided into UV-A (light wavelengths from 320 to 400 nm) and UV-B (wavelengths ranging from 280 to 320 nm) regions. Overexposure to UV-B irradiation is generally understood to lead to skin burns and erythema. In addition, overexposure to UV-A radiation may cause a loss of elasticity of the skin and the appearance of wrinkles, promoting premature skin aging. Such irradiation promotes triggering of the erythemal reaction or amplifies this reaction in certain individuals and may even be the source of phototoxic or photoallergic reactions. It is increasingly believed that overexposure to UV-A may also lead to melanoma. Thus, the application of the compositions of the invention to the skin and/or hair of an individual will provide enhanced UVR photoprotection (UV-A and/or UV-B) of the skin and/or hair of the individual.

The compositions of the invention are intended to provide a sun protection factor (SPF) rating of at least 2, with additional preferable embodiments having a sun protection factor of at least 5, at least 10, at least 15, at least 20, at least 25, at least 30, at least 35, at least 40, at least 45, at least 50, and at least 55.

The invention will be further described by means of the following examples, which are not intended to limit the invention, as defined by the appended claims, in any manner.

### EXAMPLES

### Formulation of Sunscreen Lotions

Several oil-in-water formulations comprising the sunscreen active compounds amiloxate, octinoxate, and octocrylene, individually and in various combinations, were tested in *in vitro* SPF experiments. Tables 1 and 2 list the ingredients for the formulations containing either one or two sunscreen actives used for comparative purposes. Table 3 contains ingredients for formulations that contain combinations of all three sunscreen actives, which illustrate the surprising synergy between amiloxate, octinoxate, and octocrylene. All formulations were prepared according to the following general method:
1. Keltrol and Veegum were added to the water of Part A in a suitable container and mixed with mechanical stirrer until completely hydrated. The remainder of the Part A ingredients are then added with mixing to form the water phase and heated to 165-175 °F.
2. In a separate container all of the Part B ingredients are combined and heated to 180-185 °F.
3. The Part B ingredients are then added to the water phase of Part A, mixed well, and cooled slowly to 120-125 °F.
4. The Germaben II of Part C is then added and mixed well.
5. The formulation is then cooled to room temperature and then Part D water is added QS to weight.

**Table 1**

| **Formulations Containing Single Sunscreen Active¹** | | | | | | | |
|---|---|---|---|---|---|---|---|
| **Part** | **INCI Name** | **Trade Name** | **Ex. 1** | **Ex. 2** | **Ex. 3** | **Ex. 4** | **Ex. 5** |
| **A** | Purified Water | Water | 74.75 | 77.25 | 77.25 | 72.25 | 72.25 |
| **A** | Sodium Cetearyl Sulfate | Sodium Cetearyl Sulfate | 0.30 | 0.30 | 0.30 | 0.30 | 0.30 |
| **A** | Propylene Glycol | Propylene Glcvol | 5.00 | 5.00 | 5.00 | 5.00 | 5.00 |
| **A** | Magnesium Aluminum Silicate | Veegum Ultra | 1.00 | 1.00 | 1.00 | 1.00 | 1.00 |
| **A** | Xanthan Gum | Keltrol CGF | 0.35 | 0.35 | 0.35 | 0.35 | 0.35 |
| **B** | Neopentyl Glycol Diheptanoate | Lexfeel 7 | 5.00 | 5.00 | 5.00 | 5.00 | 5.00 |
| **B** | PVP Eicosene Copolymer | Ganex V-220 | 2.00 | 2.00 | 2.00 | 2.00 | 2.00 |
| **B** | Arachidyl Alcohol (and) Behenyl Alcohol (and) Arachidyl Glucoside | Montanov 202 | 2.00 | 2.00 | 2.00 | 2.00 | 2.00 |
| **B** | Octinoxate | Parsol MCX | 7.50 | 0.00 | 0.00 | 0.00 | 0.00 |
| **B** | Amiloxate | Neoheliopan E-1000 | 0.00 | 5.00 | 0.00 | 0.00 | 10.00 |
| **B** | Octocrylene | Uvinul N 539 T | 0.00 | 0.00 | 5.00 | 10.00 | 0.00 |
| **B** | Peg-30 Dipolyhydroxystearate | Arlacel P 135 | 0.10 | 0.10 | 0.10 | 0.10 | 0.10 |
| **B** | Dimethicone | Dow Coming 200 Fluid, 350 CST | 1.00 | 1.00 | 1.00 | 1.00 | 1.00 |
| **C** | Propylene glycol (and) Diazolidinyl urea (and) methyl paraben (and) propyl paraben | Germaben II | 1.00 | 1.00 | 1.00 | 1.00 | 1.00 |
| **D** | Purified Water | Water | QS | QS | QS | QS | QD |
| | | **Total** | 100.00 | 100.00 | 100.00 | 100.00 | 100.00 |

| | | | | | | | |
|---|---|---|---|---|---|---|---|
| ¹ All amounts listed as percent by weight. | | | | | | | |

**Table 2**

| **Formulations Containing Two Sunscreen Actives¹** | | | | | | |
|---|---|---|---|---|---|---|
| **Part** | **INCI Name** | **Trade Name** | **Ex. 6** | **Ex. 7** | **Ex. 8** | **Ex. 9** |
| **A** | Purified Water | Water | 69.75 | 62.25 | 67.25 | 64.75 |
| **A** | Sodium Cetearyl Sulfate | Sodium Cetearyl Sulfate | 0.30 | 0.30 | 0.30 | 0.30 |
| **A** | Propylene Glycol | Propylene Glcyol | 5.00 | 5.00 | 5.00 | 5.00 |
| **A** | Magnesium Aluminum Silicate | Veegum Ultra | 1.00 | 1.00 | 1.00 | 1.00 |
| **A** | Xanthan Gum | Keltrol CGF | 0.35 | 0.35 | 0.35 | 0.35 |
| **B** | Neopentyl Glycol Diheptanoate | Loxfeel 7 | 5.00 | 5.00 | 5.00 | 5.00 |
| **B** | PVP Eicosene Copolymer | Ganex V-220 | 2.00 | 2.00 | 2.00 | 2.00 |
| **B** | Arachidyl Alcohol (and) Behenyl Alcohol (and) Arachidyl Glucoside | Montanov 202 | 2.00 | 2.00 | 2.00 | 2.00 |
| **B** | Octinoxate | Parsol MCX | 7.50 | 0.00 | 0.00 | 7.50 |
| **B** | Amiloxate | Neoheliopan E-1000 | 5.00 | 10.00 | 5.00 | 0.00 |
| **B** | Octocrylene | Uvinul N 539 T | 0.00 | 10.00 | 10.00 | 10.00 |
| **B** | Peg-30 Dipolyhydroxystearate | Arlacel P 135 | 0.10 | 0.10 | 0.10 | 0.10 |
| **B** | Dimethicone | Dow Corning 200 Fluid, 350 CST | 1.00 | 1.00 | 1.00 | 1.00 |
| **C** | Propylene glycol (and) Diazolidinyl urea (and) methyl paraben (and) propyl paraben | Germaben It | 1.00 | 1.00 | 1.00 | 1.00 |
| **D** | Purified Water | Water | QS | QS | QS | QS |
| | | **Total** | 100.00 | 100.00 | 100.00 | 100.00 |

| | | | | | | |
|---|---|---|---|---|---|---|
| ¹ All amounts listed as percent by weight. | | | | | | |

**Table 3**

| **Formulations Containing Three Sunscreen Actives¹** | | | | | |
|---|---|---|---|---|---|
| **Part** | **INCI Name** | **Trade Name** | **Ex. 10** | **Ex. 11** | **Ex. 12** |
| **A** | Purified Water | Water | 64.75 | 59.25 | 59.75 |
| **A** | Sodium Cetearyl Sulfate | Sodium Cetearyl Sulfate | 0.30 | 0.30 | 0.30 |
| **A** | Propylene Glycol | Propylene Glcyol | 5.00 | 5.00 | 5.00 |
| **A** | Magnesium Aluminum Silicate | Veegum Ultra | 1.00 | 1.00 | 1.00 |
| **A** | Xanthan Gum | Keltrol CGF | 0.35 | 0.35 | 0.35 |
| **B** | Neopentyl Glycol Diheptanoate | Lexfeel 7 | 5.00 | 5.00 | 0.00 |
| **B** | PVP Eicosene Copolymer | Ganex V-220 | 2.00 | 2.00 | 2.00 |
| **B** | Arachidyl Alcohol (and) Behenyl Alcohol (and) Arachidyl Glucoside | Montanov 202 | 2.00 | 2.00 | 2.00 |
| **B** | Octinoxate | Parsol MCX | 7.50 | 7.50 | 7.50 |
| **B** | Amiloxate | Neoheliopan E-1000 | 5.00 | 5.00 | 10.00 |
| **B** | Octocrylene | Uvinul N 539 T | 5.00 | 10.00 | 10.00 |
| **B** | Peg-30 Dipolyhydroxystearate | Arlacel P 135 | 0.10 | 0.10 | 0.10 |
| **B** | Dimethicone | Dow Coming 200 Fluid, 350 CST | 1.00 | 1.00 | 1.00 |
| **C** | Propylene glycol (and) Diazolidinyl urea (and) methyl paraben (and) propyl paraben | Germaben II | 1.00 | 1.00 | 1.00 |
| **D** | Purified Water | Water | QS | QS | QS |
| | | **Total** | 100.00 | 100.00 | 100.00 |

| | | | | | |
|---|---|---|---|---|---|
| ¹ All amounts listed as percent by weight. | | | | | |

### SPF Measurements

All SPF measurements were conducted using Vitro-Skin® (IMS, Inc., Orange, CT; USA) as the substrate to which the sunscreen formulas were applied. The substrates were hydrated according to the manufacturer's instructions. Test formulations were applied to the substrates at 2 mg/cm², mounted in 6X7 photographic slide mounts (B&H Photo Video, New York, NY; USA) and allowed to dry for 20 minutes. An untreated, hydrated Vitro-Skin® substrate was also mounted in a slide mount and allowed to air dry for 20 minutes to serve as a reference and blank. The in vitro SPF of each formula was determined using an Optometrics SPF 290S Analzyer (Optometrics LLC. Ayer, MA; USA) equipped with a computer-controlled X-Y sampling stage and operated according to the manufacturer's instructions. A total of 12 in vitro SPF values were obtained for each formula using a different, non-overlapping position on each mounted substrate. The resulting in vitro SPF values are shown in Table 4. All the SPF values reported are the average of 12 values measured for each formulation.

**Table 4**

| **Measured vs. Expected SPF** | | | | | |
|---|---|---|---|---|---|
| **Formula Example** | **Octinoxate (% w/v)** | **Amiloxate (%w/v)** | **Octocrylene (% w/v)** | **Measured SPF (sd)¹** | **Expected SPF²** |
| **1** | 7.5 | -- | -- | 10.5 (1.0) | |
| **2** | -- | 5.0 | -- | 8.6 (1.3) | |
| **3** | -- | -- | 5.0 | 5.6 (0.4) | |
| **4** | -- | -- | 10.0 | 15.6 (3.0) | |
| **5** | -- | 10.0 | -- | 13.1 (1.5) | |
| **6** | 7.5 | 5.0 | -- | 18.0 (1.3) | 19.1 |
| **7** | -- | 10.0 | 10.0 | 30.2 (3.7) | 28.7 |
| **8** | -- | 5.0 | 10.0 | 24.6 (3.3) | 24.2 |
| **9** | 7.5 | -- | 10.0 | 26.5 (3.9) | 26.1 |
| **10** | 7.5 | 5.0 | 5.0 | 30.0 (2.4) | 24.7 |
| **11** | 7.5 | 5.0 | 10.0 | 42.0 (5.9) | 34.7 |
| **12** | 7.5 | 10.0 | 10.0 | 48.2 (5.0) | 39.2 |

| | | | | | |
|---|---|---|---|---|---|
| ¹Measured SPF represents the mean of three different determinations. ²Expected SPF values reported for Examples 6-12 were calculated by adding the sum of the individual SPF's from each sunscreen at the same concentration. | | | | | |

The results in Table 4 clearly demonstrate that a unique synergy exists for the combination of sunscreen actives of the invention, as demonstrated for example in formulas 10-12 containing various levels of all three sunscreen actives. Formulas 10-12 increase SPF values by 5 to 9 SPF units relative to the SPF's expected from the arithmetic sum of the SPF's from individual sunscreen actives at the same concentrations. In contrast, measured SPF values of formulas 6-9 containing two sunscreen actives remain about the same as the expected SPF values, confirming that the synergies observed for the combination of all three sunscreen actives do not derive from any possible combination of the two sunscreen actives. The synergistic effect in SPF performance only exists for combinations of all three sunscreen actives in the same formula.

In addition to the oil-in-water emulsions described above, additional representative examples of photoprotective compositions according to the invention can be made for example as water-in-oil emulsions and alcohol spray compositions. Representative Examples 13 (water-in-oil emulsion) and 14 (alcohol spray) are described below.

**Table 5**

| **Water-in-Oil Emulsion Formulation (Ex. 13)** | | | | |
|---|---|---|---|---|
| **Part** | **No.** | **INCI Name** | **Trade Name** | **Wt. %(w/w)** |
| **A** | 1 | Octinoxate | Parsol MCX | 7.50 |
| **A** | 2 | Amiloxate | NeoHeliopan E-1000 | 10.00 |
| **A** | 3 | Octocrylene | Unvinul N 539 T | 10.00 |
| **A** | 4 | Bis-hydroxyethyoxypropyl Dimethicone | DC 5562 Carbinol Fluid | 4.00 |
| **A** | 5 | Cyclopentasiloxane (and) PEG-12 Dimethicone Crosspolymer | Dow Coming 9011 Silicone Elastomer | 5.00 |
| **B** | 6 | Tocopherol | d,l - alpha Tocopherol | 0.20 |
| **B** | 7 | *Purified Water* | Water | 56.70 |
| **B** | 8 | Propylene Glycol | Propylene Glycol | 5.00 |
| B | 9 | Polyaminopropyl Biguanide | Cosmocil CQ | 1.00 |
| **B** | 10 | Sodium Chloride | Sodium Chloride | 0.50 |
| **B** | 11 | Chlorphenesin | 3-(4-Chlorphenoxy)-1,2-Propanediol | 0.10 |
| | | | **Total** | 100.00 |

The water-in-oil formulation above can be prepared by first adding the oil phase ingredients of part A in a container large enough to hold the entire batch and mixing until uniform. Next, in a separate container add the water of part B followed by the remaining ingredients of Part B in the listed order and mix well. Finally, the water phase of the second step can be added slowly to the oil phase of step one with slow mixing until all of the aqueous phase has been added and then the mixing speed is increased for rapid mixing to complete the emulsification and set the lotion composition.

**Table 6**

| Alcohol **Spray Formulation (Ex. 14)** | | | | |
|---|---|---|---|---|
| **Part** | | **INCI Name** | **Trade Name** | **Wt. % (w/w)** |
| **A** | **1** | Octinoxate | Parsol MCX | 7.50 |
| **A** | **2** | Amiloxate | Neoheliopan E-1000 | 5.00 |
| **A** | **3** | Octocrylene | Uvinul N 539 T | 5.00 |
| **A** | **4** | SD Alcohol 40 | SD Alcohol 40 | 79.30 |
| **A** | **5** | Acrylates/Octylacrylamide Copolymer | Dermacryl 79 | 3.00 |
| **A** | **6** | Fragrance | Fragrance | 0.20 |
| | | | **Total** | 100.00 |

The composition is prepared by adding all ingredients to a suitable sized container in the order listed and mixing until uniform. The compositions can then be loaded, for example, into a pump bottle for spray application to the skin and/or hair.

The compositions of the invention can also be prepared as aerosol formulations for application as a spray to the skin and/or hair. The following composition in Table 7 (Example 15) is an example of the production of an aerosol formulation according to the subject invention.

**Table 7**

| **Aerosol Lotion Formulation** | | | |
|---|---|---|---|
| **Part** | **INCI Name** | **Trade Name** | **Wt. % (w/w)** |
| **A** | Purified Water | Water | 41.83 |
| **A** | Sodium Cetearyl Sulfate | Sodium Cetearyl Sulfate | 0.21 |
| **A** | Propylene Glycol | Propylene Glcyol | 3.50 |
| **A** | Magnesium Aluminum Silicate | Veegum Ultra | 0.70 |
| **A** | Xanthan Gum | Keltrol CGF | 0.24 |
| **B** | PVP Eicosene Copolymer | Ganex V-220 | 1.4 |
| **B** | Arachidyl Alcohol (and) Behenyl Alcohol (and) Arachidyl Glucoside | Montanov 202 | 1.4 |
| **B** | Octinoxate | Parsol MCX | 5.25 |
| **B** | Amiloxate | Neoheliopan E-1000 | 7.00 |
| **B** | Octocrylene | Uvinul N 539 T | 7.00 |
| **B** | Peg-30 Dipolyhydroxystearate | Arlacel P 135 | 0.07 |
| **B** | Dimethicone | Dow Corning 200 Fluid, 350 CST | 0.70 |
| **C** | Propylene glycol (and) Diazolidinyl urea (and) methyl paraben (and) propyl paraben | Germaben II | 0.70 |
| **E** | Dimethyl Ether | | 30.0 |
| | | **Total** | 100.00 |

The aerosol lotion listed in Table 7 can be easily prepared by combining the lotion formulation described herein with a common propellant and loaded into a suitable container to allow for storage until applied. In the above Example 15, 70% of the lotion described in Example 12 can be combined with a common propellant, in this case 30% dimethyl ether, in a closed container, such as an aluminum can or glass bottle.

To prepare the aerosol formulation, the emulsion is allowed to cool to room temperature and then weighed into the appropriate container such as an aluminum aerosol can or glass bottle. The appropriate valve, dip tube and actuator are placed in the can or bottle and the system is crimped and sealed using a hand crimper designed for the specific diameter of the can or bottle. The sealed assembly is then immersed in an ice bath. While the assembly is maintained at or near 0°C, the propellant is introduced into the can or bottle from a gas cylinder containing the dimethyl ether by depressing the actuator stem and allowing the gas to flow into the aerosol can or bottle. This transfer occurs because the pressure inside the aerosol can or bottle at 0°C is less than the pressure in the gas cylinder at ambient temperature. The can or bottle can be periodically weighed to determine the amount of propellant that has been added.

Although certain presently preferred embodiments of the invention have been described herein, it will be apparent to those skilled in the art to which the invention pertains that variations and modifications of the described embodiments may be made without departing from the scope of the invention, which is defined by the claims.

## Claims

1. A topically applicable sunscreen composition suited for the photoprotection of human skin and/or hair, comprising photoprotecting synergistically effective amounts of octinoxate, octocrylene, and amiloxate;
wherein octinoxate is present in the composition in an amount from about 6.5 to about 7.5 percent by weight, and octocrylene and amiloxate are each present in the composition in an amount from about 5.0 to about 10.0 percent by weight.

2. The sunscreen composition of claim 1, wherein octocrylene is present in the composition in an amount from about 8.0 to about 10.0 percent by weight.

3. The sunscreen composition of claim 1, wherein amiloxate is present in the composition in an amount from about 7.5 to about 10.0 percent by weight.

4. The sunscreen composition of claim 1, further comprising at least one additional UV-A and/or UV-B sunscreen.

5. The sunscreen composition of claim 4, wherein the additional UV-A and/or UV-B sunscreen comprises a photoprotecting effective amount of particulates of at least one inorganic pigment or nanopigment.

6. The sunscreen composition of claim 5, wherein said at least one pigment or nanopigment comprises titanium dioxide, zinc oxide, iron oxide, zirconium oxide, cerium oxide, or mixture thereof.

7. The sunscreen composition of claim 1, further comprising at least one UV-radiation scattering agent.

8. The sunscreen composition of claim 1, further comprising at least one sunless-tanning agent or self-tanning composition.

9. The sunscreen composition of claim 1, comprising a nonionic vesicle dispersion, emulsion, cream, milk, gel, cream gel, ointment, suspension, dispersion, powder, solid, stick, foam or spray.

10. The sunscreen composition of claim 1, comprising an anhydrous or aqueous solid or paste, emulsion, suspension, or dispersion.

11. The sunscreen composition as defined by claim 1, comprising an oil-in-water emulsion.

12. The sunscreen composition as defined by claim 1, comprising a water-in-oil emulsion.

13. The sunscreen composition as defined by claim 1, comprising an aerosol formulation.

14. The sunscreen composition as defined by claim 1, having a sun protection factor of at least 2.

15. The sunscreen composition as defined by claim 1, having a sun protection factor of at least 30.

16. The composition of claim 1 which is a cosmetic composition for topical application to human skin and/or hair.

17. The cosmetic composition of claim 16, wherein the composition comprises a moisturizer, cleanser, conditioner, shampoo, body wash, styling gel/lotion, eye cream, eye liner, blush, mascara, foundation, nail polish, polish remover, eye shadow, lipstick, lip gloss, lip liners, lip balms, makeup remover, nail treatment, foot care compositions, acne treatment, redness/rosacea treatment, varicose/spider vein treatment, anti-aging compositions, sunless tanning compositions, after-sun compositions, concealers, hair color and bleaching compositions, skin fading/lighteners, body firming lotion, shaving cream, after shave, relaxer, antiperspirant, deodorant, exfoliant, scrub, liquid hand soap, bubble bath, pain and wound treatment, insect repellant, anti-itch/rash cream, styling mousse, foams, perfume, lubricant, body oil, body spray, baby lotion, diaper cream, baby soap, baby shampoo, baby oil, baby wipes, hair-loss treatment, hair spray, depilatory, hair growth inhibitor, hair removal wax, personal cleansing, cologne, oil controller, or hand sanitizer.

18. The cosmetic composition of claim 16 further comprising at least one skin protectant active agent.

19. The cosmetic composition of claim 18, wherein said skin protectant active agent comprise allantoin, aluminum hydroxide gel, calamine, cocoa butter, cod liver oil, colloidal oatmeal, dimethicone, glycerin, hard fat, kaolin, lanolin, mineral oil, petrolatum, sodium bicarbonate, topical starch, white petrolatum, zinc acetate, zinc carbonate, and zinc oxide.

20. A composition of any previous claim for use in a method for protecting human skin and/or hair against the deleterious effects of solar or ultraviolet radiation.

## Patentansprüche

1. Topisch anwendbare Sonnenschutzzusammensetzung, die für den Lichtschutz von menschlicher Haut und/oder menschlichem Haar geeignet ist, umfassend lichtschützend synergistisch wirksame Mengen von Octinoxat, Octocrylen und Amiloxat;
wobei Octinoxat in einer Menge von etwa 6,5 bis etwa 7,5 Gewichtsprozent in der Zusammensetzung vorhanden ist und Octocrylen und Amiloxat jeweils in Mengen von etwa 5,0 bis etwa 10,0 Gewichtsprozent in der Zusammensetzung vorhanden sind.

2. Sonnenschutzzusammensetzung gemäß Anspruch 1, wobei Octocrylen in einer Menge von etwa 8,0 bis etwa 10,0 Gewichtsprozent in der Zusammensetzung vorhanden ist.

3. Sonnenschutzzusammensetzung gemäß Anspruch 1, wobei Amiloxat in einer Menge von etwa 7,5 bis etwa 10,0 Gewichtsprozent in der Zusammensetzung vorhanden ist.

4. Sonnenschutzzusammensetzung gemäß Anspruch 1, ferner umfassend wenigstens ein zusätzliches UV-A- und/oder UV-B-Sonnenschutzmittel.

5. Sonnenschutzzusammensetzung gemäß Anspruch 4, wobei das zusätzliche UV-A- und/oder UV-B-Sonnenschutzmittel eine lichtschützend wirksame Menge von Partikulaten von wenigstens einem anorganischen Pigment oder Nanopigment umfasst.

6. Sonnenschutzzusammensetzung gemäß Anspruch 5, wobei das wenigstens eine Pigment oder Nanopigment Titandioxid, Zinkoxid, Eisenoxid, Zirkoniumoxid, Ceroxid oder ein Gemisch davon umfasst.

7. Sonnenschutzzusammensetzung gemäß Anspruch 1, ferner umfassend wenigstens ein UV-Strahlenstreuendes Mittel.

8. Sonnenschutzzusammensetzung gemäß Anspruch 1, ferner umfassend wenigstens ein Mittel zum sonnenlosen Bräunen oder eine Selbstbräunungszusammensetzung.

9. Sonnenschutzzusammensetzung gemäß Anspruch 1, umfassend eine nichtionische Vesikeldispersion, Emulsion, Creme, Milch, ein Gel, eine Gelcreme, Salbe, Suspension, Dispersion, ein Pulver, einen Feststoff, Stift, Schaum oder ein Spray.

10. Sonnenschutzzusammensetzung gemäß Anspruch 1, umfassend eine(n) wasserfreie(n) oder wasserhaltige(n) Feststoff oder Paste, Emulsion, Suspension oder Dispersion.

11. Sonnenschutzzusammensetzung gemäß Anspruch 1, umfassend eine Öl-in-Wasser-Emulsion.

12. Sonnenschutzzusammensetzung gemäß Anspruch 1, umfassend eine Wasser-in-Öl-Emulsion.

13. Sonnenschutzzusammensetzung gemäß Anspruch 1, umfassend eine Aerosolformulierung.

14. Sonnenschutzzusammensetzung gemäß Anspruch 1 mit einem Sonnenschutzfaktor von wenigstens 2.

15. Sonnenschutzzusammensetzung gemäß Anspruch 1 mit einem Sonnenschutzfaktor von wenigstens 30.

16. Sonnenschutzzusammensetzung gemäß Anspruch 1, die eine kosmetische Zusammensetzung für die topische Anwendung auf menschliche(s) Haut und/oder Haar ist.

17. Kosmetische Zusammensetzung gemäß Anspruch 16, wobei die Zusammensetzung ein/eine/einen Feuchthaltemittel, Reinigungsmittel, Konditionierungsmittel, Shampoo, Body-Wash, Styling-Gel/Lotion, Augencreme, Eyeliner, Rouge, Maskara, Make-up, Nagellack, Nagellackentferner, Lidschatten, Lippenstift, Lipgloss, Lippenkonturenstift, Lippenbalsam, Make-up-Entferner, Nagelbehandlungsmittel, Fußpflegezusammensetzung, Aknebehandlungsmittel, Rötungs/Rosazea-Behandlungsmittel, Varizen/Besenreiser-Behandlungsmittel, Anti-Aging-Zusammensetzung, Zusammensetzung zum sonnenfreien Bräunen, After-Sun-Zusammensetzung, Abdecker, Haarfärbe- und Bleichzusammensetzung, Hautbleicher/-aufheller, Körperstraffungslotion, Rasiercreme, Aftershave, Entspannungsmittel, Antitranspirant, Deodorant, Exfoliant, Reiniger, flüssige Handseife, Schaumbad, Schmerz- und Wundbehandlungsmittel, Insektenabwehrmittel, Anti-Juckreiz/Ausschlag-Creme, Styling-Mousse, Schaum, Parfüm, Gleitmittel, Körperöl, Körperspray, Babylotion, Windelcreme, Babyseife, Babyshampoo, Babyöl, Babytuch, Haarausfallbehandlungsmittel, Haarspray, Enthaarungsmittel, Haarwachstumshemmer, Haarentfernungswachs, Körperreinigungsmittel, Kölnischwasser, Ölregulierungsmittel oder Handdesinfektionsmittel umfasst.

18. Kosmetische Zusammensetzung gemäß Anspruch 16, ferner umfassend wenigstens einen Hautschutzwirkstoff.

19. Kosmetische Zusammensetzung gemäß Anspruch 18, wobei der Hautschutzwirkstoff Allantoin, Aluminiumhydroxid-Gel, Calamin, Kakobutter, Dorschleberöl, kolloidales Hafermehl, Dimethicon, Glycerin, Hartfett, Kaolin, Lanolin, Mineralöl, Petrolatum, Natriumbicarbonat, topische Stärke, weißes Petrolatum, Zinkacetat, Zinkcarbonat und Zinkoxid umfasst.

20. Zusammensetzung gemäß einem der vorstehenden Ansprüche für die Verwendung bei einem Verfahren zum Schützen von menschlicher Haut und/oder menschlichem Haar gegen die schädlichen Wirkungen von Sonnen- oder Ultraviolettstrahlung.

## Revendications

1. Composition d'écran solaire applicable par voie topique, appropriée pour la photo-protection de la peau et/ou des cheveux humains, comprenant des quantités photo-protectrices et efficaces sur le plan synergique d'octinoxate, d'octocrylène et d'amiloxate;
où l'octinoxate est présent dans la composition selon une quantité allant d'environ 6,5 à environ 7,5% en poids, et l'octocrylène et l'amiloxate sont chacun présents dans la composition selon une quantité allant d'environ 5,0 à environ 10,0% en poids.

2. Composition d'écran solaire selon la revendication 1, où l'octocrylène est présent dans la composition selon une quantité allant d'environ 8,0 à environ 10,0% en poids.

3. Composition d'écran solaire selon la revendication 1, où l'amiloxate est présent dans la composition selon une quantité allant d'environ 7,5 à environ 10,0% en poids.

4. Composition d'écran solaire selon la revendication 1, comprenant en outre au moins un autre écran solaire anti-UVA et/ou UVB.

5. Composition d'écran solaire selon la revendication 4, où l'autre écran solaire anti-UVA et/ou UVB comprend une quantité efficace photo-protectrice de substances particulaires d'au moins un pigment ou nano-pigment inorganique.

6. Composition d'écran solaire selon la revendication 5, où ledit au moins un pigment ou nano-pigment comprend du dioxyde de titane, de l'oxyde de zinc, de l'oxyde de fer, de l'oxyde de zirconium, de l'oxyde de cérium, ou un mélange de ceux-ci.

7. Composition d'écran solaire selon la revendication 1, comprenant en outre au moins un agent de diffusion du rayonnement UV.

8. Composition d'écran solaire selon la revendication 1, comprenant en outre au moins un agent auto-bronzant ou une composition auto-bronzante.

9. Composition d'écran solaire selon la revendication 1, comprenant une dispersion de vésicules non ioniques, une émulsion, une crème, un lait, un gel, un gel-crème, un onguent, une suspension, une dispersion, une poudre, un solide, un bâtonnet, une mousse ou un spray.

10. Composition d'écran solaire selon la revendication 1, comprenant une pâte ou un solide anhydre ou aqueux ou une émulsion, une suspension ou une dispersion.

11. Composition d'écran solaire telle que définie selon la revendication 1, comprenant une émulsion huile-dans-eau.

12. Composition d'écran solaire telle que définie selon la revendication 1, comprenant une émulsion eau-dans-huile.

13. Composition d'écran solaire telle que définie selon la revendication 1, comprenant une formulation d'aérosol.

14. Composition d'écran solaire telle que définie selon la revendication 1, ayant un facteur de protection solaire d'au moins 2.

15. Composition d'écran solaire telle que définie selon la revendication 1, ayant un facteur de protection solaire d'au moins 30.

16. Composition selon la revendication 1, qui est une composition cosmétique destinée à une application topique à la peau et/ou aux cheveux humains.

17. Composition cosmétique selon la revendication 16, où la composition comprend un hydratant, un nettoyant, un après-shampooing, un shampooing, un nettoyant pour le corps, un gel/une lotion de coiffage, une crème pour les yeux, un eye-liner, un fard à joues, un mascara, un fond de teint, un vernis à ongles, un dissolvant à ongles, un fard à paupières, un rouge à lèvres, un brillant à lèvres, un crayon contour des lèvres, un baume à lèvres, un démaquillant, un traitement pour les ongles, des compositions de soin des pieds, un traitement pour l'acné, un traitement pour les rougeurs/acné rosacée, un traitement pour les varices/ télangiectasies, des compositions antivieillissement, des compositions auto-bronzantes, des compositions après-soleil, des correcteurs de teint, des compositions de coloration et de décoloration des cheveux, des blanchissants/éclaircissants de la peau, une lotion de raffermissement du corps, une crème à raser, un after-shave, un relaxant, un anti-transpirant, un déodorant, un exfoliant, un soin gommant, un savon liquide pour les mains, un bain moussant, un traitement pour les douleurs et les plaies, un insectifuge, une crème anti-démangeaisons/ rougeurs, une mousse coiffante, des mousses, un parfum, un lubrifiant, une huile pour le corps, un spray pour le corps, une lotion pour bébé, une crème pour érythème fessier, un savon pour bébé, un shampooing pour bébé, une huile pour bébé, des lingettes pour bébé, un traitement contre la chute des cheveux, un spray capillaire, un dépilatoire, un inhibiteur de la croissance des poils, une cire dépilatoire, un nettoyant personnel, une eau de Cologne, un anti-sébum, ou un assainisseur pour les mains.

18. Composition cosmétique selon la revendication 16, comprenant en outre au moins un agent actif de protection de la peau.

19. Composition cosmétique selon la revendication 18, où ledit agent actif de protection de la peau comprend de l'allantoïne, un gel à base d'hydroxyde d'aluminium, de la calamine, du beurre de cacao, de l'huile de foie de morue, de la farine d'avoine colloïdale, de la diméthicone, du glycérol, une matière grasse dure, du kaolin, de la lanoline, de l'huile minérale, du pétrolatum, du bicarbonate de sodium, de l'amidon topique, de la vaseline blanche, de l'acétate de zinc, du carbonate de zinc, et de l'oxyde de zinc.

20. Composition selon l'une quelconque des revendications précédentes, pour une utilisation dans une méthode de protection de la peau et/ou des cheveux humains contre les effets délétères du rayonnement solaire ou ultraviolet.
